# EUROPÄISCHE PATENTSCHRIFT

(11) **EP 1 187 594 B1**
(45) Veröffentlichungstag und Bekanntmachung des Hinweises auf die Patenterteilung: **29.12.2010**
(21) Anmeldenummer: 00987236.7
(22) Anmeldetag: 17.11.2000
(51) Int. Cl.: A61K 6/00

(54) **OBERFLÄCHENBEHANDLUNGSMITTEL FÜR DENTALES HARTGEWEBE**
SURFACE TREATMENT AGENT FOR HARD DENTAL TISSUE
AGENTS DE TRAITEMENT SUPERFICIEL POUR TISSUS DENTAIRES DURS

(30) Priorität: 19.11.1999 DE 19955746
(43) Veröffentlichungstag der Anmeldung: 20.03.2002
(73) Patentinhaber: 3M ESPE AG, 82229 Seefeld (DE)
(72) Erfinder: HÄBERLEIN, Ingo, 82362 Weilheim (DE); LUCHTERHANDT, Thomas, 86926 Greifenberg (DE); HECHT, Reinhold, 86916 Kaufering (DE); FREY, Oliver, 82131 Gauting-Königswiesen (DE)
(86) Internationale Anmeldenummer: PCT/EP2000/011187
(87) Internationale Veröffentlichungsnummer: WO 2001/037787

(56) Entgegenhaltungen:
- WO-A-98/29088
- US-A- 5 762 502
- YOHSUKE T ET AL: "A study on cytochrome c oxidoreductase for bonding a tri-n-butylborane-initiated luting agent to dentin" JOURNAL OF BIOMEDICAL MATERIALS RESEARCH, Bd. 48, Nr. 5, Mai 1999 (1999-05), Seiten 697-699, XP000997686
- DATABASE WPI Section Ch, Week 197630 Derwent Publications Ltd., London, GB; Class A96, AN 1976-56868X XP002164706 & JP 51 067346 A (LION DENTIFRICE CO LTD), 10. Juni 1976 (1976-06-10)

## Beschreibung

Die vorliegende Erfindung betrifft ein Verfahren zur Verbesserung der adhäsiven Befestigung von Dentalmaterialien auf der Zahnhartsubstanz durch Modifizierung der nach der Präparation bzw. dem Ätzen vorhandenen Kollagenschicht.

Zur Befestigung von Dentalmaterialien auf der präparierten Zahnhartsubstanz werden nach heutigem Stand der Technik durch den Behandler folgende Arbeitsschritte durchlaufen:
1. Anätzen der gesamten Zahnhartsubstanz durch eine Säure ("total-etch-Technik").
2. Auftragen eines sog. Primers, der oberflächlich in die Zahnhartsubstanz einpenetriert.
3. Auftragen eines sog. Bondings, welches zusammen mit dem Primer eine Hybridschicht bildet.
4. Polymerisation des Bondings (z.B. durch Bestrahlen mit Licht und/oder durch Redox-Reaktion).
5. Aufbringen des Dentalmaterials.

Um die Anzahl der anzuwendenden Bestandteile zu verringern, wurden der Primer und das Bonding - oder auch Versiegler - zu einer Komponente zusammengefaßt (sog. Einflaschen-Bondings, z.B. Prime & Bond 2.1, Fa. Dentsply/Detray, Dreieich). Dennoch muss zuerst geätzt und gespült werden, um die bei der Präparation entstandene Schmierschicht - ein Gemisch, das im wesentlichen aus Hydroxylapatit und Kollagen besteht - von Hydroxylapatit zu befreien, damit anschließend das Einflaschen-Bonding mindestens einmal aufgetragen und anschließend polymerisiert werden kann, bevor das Dentalmaterial zur Anwendung kommt. In der Literatur finden sich Hinweise darauf, dass die Effizienz dieser vereinfacht anzuwendenden Adhäsive in Frage zu stellen ist (R. Frankenberger, N. Krämer, J. Sindel; Dtsch. Zahnärztl. Z, 51, (1996), 556-560). Insbesondere die Lebensdauer des Haftverbundes nimmt bei diesem vereinfachten Vorgehen stark ab.

Eine andere Vereinfachung des oben beschriebenen Verfahrens zur adhäsiven Befestigung von Dentalmaterialien besteht darin, den Primer und das Ätzmittel zu einer Komponente zu kombinieren (sog. selbstätzende Primer; z.B. Etch & Prime 3.0, Fa. Degussa; Clearfill-Liner Bond 2.0, Fa. Kuraray, Osaka). Diese müssen nur noch aufgetragen und nicht mehr abgespült werden - sogenanntes "Modifizieren der Schmierschicht" - , ihnen folgt entweder das zu polymerisierende Bonding (z.B. Clearfill-Liner Bond 2.0, Fa. Kuraray, Osaka) oder zumindest aber ein Vernetzungsschritt zur Polymerisation des selbstätzenden Primers, bevor das Dentalmaterial verwendet werden kann.

Nach dem ersten Arbeitsschritt (Ätzung der Zahnhartsubstanz) liegt auf dem Dentin ein Netzwerk von freigelegten Kollagenfasem vor. Durch die folgende Applikation des Primers bzw. des Bondes (Einflaschen-Variante) entsteht nach erfolgter Polymerisation, initiiert durch die Belichtung und/oder Redoxreaktion, eine sog. Hybridschicht. Diese Hybridschicht besteht aus Kollagenfasern, die zusammen mit den ausgehärteten Monomeren des Primers und/oder Bondes ein interpenetrierendes Netzwerk bilden. Nach gängiger Lehrmeinung ist diese Hybridschicht hauptverantwortlich für die Haftung zwischen Zahnhartsubstanz und Dentalmaterialien (N. Nakabayashi et.al., J. Biomed. Mater. Res., 1982, 16, 265-273; R.L. Erikson, Am. J. Dent. 1989, 2, 117-123; D.H. Pashley, Trans. Acad. Dent. Mater., 1990, 3, 55-73; B.v. Meerbeck, Promotion 1993, Katolieke Universiteit te Leuven). Des weiteren wirkt die Hybridschicht als elastischer "Puffer" zwischen Zahnhartsubstanz und Dentalmaterial, da das Elastizitätsmodul in etwa zwischen beiden Materialien liegt. Insgesamt ist die Hybridschicht also ein wesentlicher Bestandteil, der zum Erfolg der Präparation und Gesundheit der verbleibenden Zahnhartsubstanz entscheidend beiträgt.

Es gibt auch Untersuchungen, die zeigen, dass nicht imprägnierte Teilbereiche der Kollagenschicht die Verbindung zwischen Zahnhartsubstanz und Dentalmaterial schwächen und möglicherweise zu einem beschleunigten Nachlassen dieser Verbindung führen (J.D. Eick, Proc. Fin. Dent. Soc., 1992, 88 (1 Suppl.), 225-242; N. Nakabayashi et.al; Dent. Mater., 1992, 8, 125-130).

Um eine gute Hybridschicht am Dentin zu erreichen, ist eine vollständige Benetzung aller Kollagenfasem und damit die Beachtung und pedantische Einhaltung der Herstellervorschriften notwendig (B.v. Meerbeck et.al., Dtsch. Zahnärztl. Z. 1994, 49, 977-984). Die Benetzung der Kollagenfasem mit den verschiedenen Bondingmaterialien ist aber von verschiedenem Faktoren, wie beispielsweise der Dicke und Trockenheit der Kollagenschicht, der Faserlänge und Hydrophilie der Bonding-Monomermatrix abhängig. Hinzu kommt, dass der Kollagenanteil in der Zahnhartsubstanz und je nach individueller Charakteristik des Patienten unterschiedlich bzw. variabel ist. Damit ist die Herstellung eines guten Haftverbundes in der zahnärztlichen Praxis eine extrem technik- und substanzsensitive Arbeit, die nur selten zu vorhersagbaren Ergebnissen führt (J. Perdigao et.al., Dent.Mater., 1997, 13, 218-227; B.v. Meerbeck et.al., Philip Journal, 1997, 9-10, 313-315).

Die US-A-5 762 502 beschreibt eine Methode bei der entweder die nach der Präparation entstandene Schmierschicht durch Behandlung mit Metalloproteinasen oder Kollagenasen entfernt werden kann (Ersatz des Ätzschrittes) oder aber nach dem Ätzen das freiliegende Kollagen durch diese Enzyme vollständig entfernt wird.

Im Journal of Biomedical Materials Research Bd. 48, Nr. 5 (1999-05), Seiten 697-699 wird eine Studie beschrieben, die sich mit Cytochrome C Oxidoreduktasen zur Befestigung eines bestimmten Befestigungszements auf Dentin befasst.

Die JP 51 067346 befasst sich mit einem dentalen Behandlungsmittel umfassend ein Zahnstein auflösendes Enzym (z.B. Dextranase) und ein hochmolekulares Material (z.B. Methyl-Methacrylat), wobei das Behandlungsmittel auch als Füllmaterial oder Beschichtungsmittel eingesetzt werden kann.

In WO 98/29088 wird ein Verfahren und Produkt zur Reinigung und/oder Bleichen von Zähnen beschrieben, welches eine Cystein-Proteinase und gegebenenfalls ein die Cystein-Protease-Aktivität blockierendes Mittel enthält.

Aufgabe der vorliegenden Erfindung ist, es die aus dem Stand der Technik bekannten Probleme zu vermeiden und ein verbessertes Oberflächenbehandlungsmittel für Hartgewebe bereitzustellen.

Gelöst wird die Aufgabe durch Bereitstellung eines Oberflächenbehandlungsmittels für Hartgewebe, umfassend mindestens ein Enzym und ein die Enzymaktivität begrenzendes Mittel wie es in den Ansprüchen beschrieben ist. Bei Anwendung des Mittel verbleibt dieses auf dem Hartgewebe, wobei die Enzymaktivität zeitlich und/oder örtlich begrenzt wird.

In Abhängigkeit von der konkreten Ausführungsform wird das Oberflächenbehandlungsmittel der vorliegenden Erfindung erst durch Mischen der einzelnen Bestandteile, insbesondere des Enzyms und des die Enzymaktivität begrenzenden Mittels, unmittelbar vor der Anwendung bzw. beim Auftragen der einzelnen Bestandteile auf die zu behandelnde Oberfläche gebildet.

Mit den Begriffen "umfassen" oder "enthalten" wird eine nicht abschließende Aufzählung von Merkmalen eingeleitet. Der Umstand, dass in den Ansprüchen das Wort "ein" vor Nennung eines Merkmals verwendet wird, schließt nicht aus, dass die genannten Merkmale mehrmals vorhanden sein können, im Sinne von "mindestens ein".

Die erfindungsgemäßen Oberflächenbehandlungsmittel können beispielsweise in Bondingformulierungen in Kombination mit einem aktivitätsterminierenden Mechanismus eingesetzt werden, so dass das oder die Enzyme auf der Zahnhartsubstanz verbleiben können, ohne diese zu schädigen.

Die erfindungsgemäßen Oberflächenbehandlungsmittel weisen unter anderem die folgende Vorteile auf:
Im Gegensatz zum Stand der Technik muss die Enzymlösung nicht jedesmal separat aufgetragen und abgespült werden. Dadurch werden dem Zahnarzt und dem Patienten drei zusätzliche Schritte (Auftragen einer Enzymlösung, Einwirken und anschließendes Abspülen) erspart, die Behandlung verkürzt und vereinfacht (anerkanntermaßen führt die Komplexität der klassischen vier Schritt Bonding Prozedur des öfteren zu Fehlern und schlechten klinischen Ergebnissen).

Ein weiterer Vorteil gegenüber dem Stand der Technik ist das Behalten der, für die Versiegelung und für die Haftung an der Zahnhartsubstanz wichtigen Hybridschicht, die auch noch eine wesentliche Funktion als elastischer Puffer zwischen Zahn und Dentalmaterial hat. Dies muss man sich dergestalt vorstellen, dass der Aufbau der Schichten mineralisiertes Dentin - Hybridschicht - Bonding - Dentalmaterial über einen Härtegradienten erfolgt, bei dem die Hybridschicht die wesentlich "Vermittlerrolle" übernommen hat.

Schließlich besteht bei den erfindungsgemäßen Oberflächenbehandlungsmitteln nun nicht mehr die Gefahr, dass bei unvollständigem Abspülen der verwendeten Enzymlösung diese zumindest teilweise auf dem Zahn verbleibt und dort - auch unter dem aufgebrachten Bonding - die Zahnhartsubstanz durch Abbau des vorhandenen Kollagens im angrenzenden Dentin schädigt.

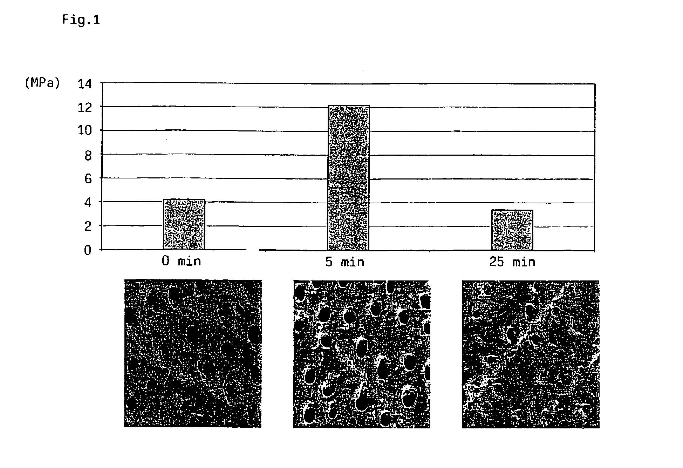

Dass durch zu langen Verbleib von Kollagenase auf freigelegtem Dentin der Verbund tatsächlich stark geschädigt wird, ist in der vorliegenden Erfindung durch die Vergleichsbeispiele und Abbildung 1 gezeigt.

Die Erfindung stellt ferner eine Methode zur Verfügung, bei der einerseits die Bondingprozedur des Standes der Technik vereinfacht und verkürzt wird, und andererseits eine wesentliche Verbesserung von Haftwerten, Techniksensitivität und Anfließverhalten an die mineralisierte Dentinoberfläche unter Beibehaltung der Hybridschicht erreicht wird.

Durch das Zerkleinern oder Modifizieren der Kollagenfasern durch Proteasen, erreicht man eine gleichmäßigere und immer gleiche Gestaltung der zu benetzenden Oberflächen. Da der Diffusionswiderstand der Monomermoleküle des Primers bzw. Bondings gegen zerkleinerte oder modifizierte Kollagenfasem wesentlich geringer ist, kann die Ausbildung einer guten Hybridschicht schneller und vollständiger erfolgen. Es ergibt sich ein immer gleiches und verbessertes Anfließverhalten, was eine vollständige Benetzung der mineralisierten Dentinoberfläche und damit auch eine geringere Sensitivität der Bondingtechnik zur Folge hat. In Konsequenz erhält man eine Verbesserung der klinischen Sicherheit der gesamten Füllung bei verbesserter Haftung. Des weiteren wird der Erhalt der Hybridschicht, mit all ihren oben genannten Vorteilen gewährleistet.

Durch die erfindungsgemäße Verwendung der Enzyme lassen sich die Haftwerte von Füllungsmaterialien an Zähnen bzw. dem Bonding erhöhen und die Standardabweichung bei den Messungen erniedrigen, wodurch die Bereitstellung von Formulierungen möglich wird, die reproduzierbarere Ergebnisse liefern.

Ein weiterer Vorteil der Erfindung liegt in dem verkürzten Verfahren, das den Einsatz der Enzyme direkt in den Bonding- oder Versieglermaterialien ermöglicht und damit dem behandelndem Arzt hilft, möglich Fehler zu vermeiden und für den Patienten die Behandiungszeit verkürzt.

Im folgenden wird die Erfindung näher beschrieben, wobei der Begriff Enzym sowohl proteolytische Enzyme, als auch Proteasen umfasst.

Als Proteasen kommen prinzipiell solche in Betracht, die fähig sind Kollagenfasern, wie sie im Zahnhartmaterial vorkommen, zu zerschneiden, und/oder solche, die fähig sind, die Kollagenfasem so zu verändern, dass diese ein verbessertes Löslichkeitsverhalten zeigen. Unter dem Begriff Proteasen gruppieren sich all jene Enzyme, die Proteine proteolytisch zu verändern vermögen. Zu den Proteasen gehören z.B. Peptidasen, Peptidylpeptidasen, Dipeptidasen, Dipeptidylpeptidasen, Oligopeptidasen, Proteinasen, Endopeptidasen, Exopeptidasen. Eine Klassifizierung von Proteasen kann auch hinsichtlich der an der proteolytischen Katalyse beteiligten Aminosäuren oder Cofaktoren erfolgen. So unterscheidet man zwischen Serinpeptidasen, Cysteinpeptidasen, Aspartatpeptidasen und Metallopeptidasen und deren Unterklassen. Eine aktuelle Übersicht an proteolytisch wirksamen Enzymen wird in A. J. Barrett, N. D. Rawlings, J. F. Woessner, Handbook of Proteolytic Enzymes, Academic Press, San Diego, 1998 gegeben, in der auch Proteasen genannt sind, die bisher noch nicht bestimmten Proteaseklassen zugeordnet werden konnten. Diese Aufzählung der erfindungsgemäßen Proteasen ist beispielhaft und in keiner Weise limitierend im Sinne der Erfindung zu verstehen. Üblicherweise verfügen Proteasen über definierte Wirkungsorte. So werden je nach Protease nur definierte Bereiche der Kollagenfasem angegriffen. Proteasen ermöglichen somit eine kontrollierte ortsspezifische Verwendung. Darüber hinaus kann die Aktivität von Proteasen zeitlich zielgerichtet gesteuert werden, wohingegen die chemische Reaktion der chemischen Agentien bis zum vollständigen Verbrauch abläuft.

Proteasen können im Sinne der Erfindung einzeln oder in Kombination mit anderen Proteasen eingesetzt werden.

Für den Einsatz der Enzyme kommen prinzipiell die folgenden Möglichkeiten und deren Kombinationen in Betracht:
- In einem separat aufgetragenen Conditioner, der üblicherweise neutral, ggf. auch sauer oder basisch sein kann;
- In einem üblichen für Bondings verwendeten Primer;
- In einem selbstätzenden Primer;
- In einem sogenannten One-bottel-bond;
- In einem selbstätzenden Bonding, sogenannten one-step bond oder auch all-inone-adhäsiv;

Zusätzlich zu Enzymen können auch chemische Agentien eingesetzt werden, die Proteine zerschneiden oder in der Struktur zu verändern vermögen. Beispielhaft seien N-halogenierte Amine, Hypochlorit, H₂O₂ genannt.

Das Oberflächenbehandlungsmittel enthält gegebenenfalls ferner eine oder mehrere Substanzen gewählt aus: Füllstoffen, insbesondere Permanent-Füllstoffen, Farbstoffen, Fließmodifikatoren, Stabilisatoren, Lösungsmittel, ionenabgebende Substanzen, bakterizid oder antibiotisch wirksame Substanzen, die Röntgenopazität erhöhende Verbindungen oder weitere Modifikatoren.

Geeignete Initiatoren umfassen Campherchinon in Kombination mit Aminen (aromtische Amine), 1,2-Diketone und (Mono, Bis und Tris)-Acylphosphinoxide. Geeignete radikalbildende Initiatoren sind in der Literatur beschrieben (z. B. J.-P Fouassier, Photoinitiation, Photopolymerization and Photocuring, Hanser Publishers, Munich, Vienna, New York, 1995 oder auch J.-P Fouassier, J. F. Rabek (Hrsg.), Radiation Curing in Polymer Science and Technology, Vol. II, Elsevier Apllied Science,, London, New York, 1993). Sie können durch UV- oder sichtbares Licht aktivierbare Substanzen sein, wie Benzoinalkylether, Benzilketale, Acylphosphinoxide oder aliphatische und aromatische 1,2-Diketonverbindungen, beispielsweise Campherchinon, wobei die Katalysatoraktivität durch Zusatz von Aktivatoren, wie tertiären Aminen oder organischen Phosphiten, in an sich bekannter Weise beschleunigt werden kann.

Geeignete Initiatorsysteme zur Auslösung der radikalischen Polymerisation über einen Redox-Mechanismus sind beispielsweise die Systeme Peroxid/Amin, Peroxid/Barbitursäurederivate oder Peroxid/Säuren u. dergl. Bei Verwendung solcher Initiatorsysteme ist es zweckmäßig, einen Initiator (z. B. Peroxid) und eine Katalysatorkomponente (z. B. Amin) getrennt bereitzuhalten. Die beiden Komponenten werden dann kurz vor ihrer Anwendung miteinander homogen gemischt.

Polymerisierbare Substanzen umfassen insbesondere mono- oder polyfunktionelle Acrylate und Methacrylate, wie sie in der EP-A-0 480 472 beschrieben sind. Ebenfalls verwendbar sind funktionalisierte Monomere mit endständigen Acrylat-oder Methacrylatgruppen, wie sie in der DE-A-2 312 559 und der EP-A-0 219 058 beschrieben sind. Typische Vertreter dieser Verbindungs-klasse (DE-A-4 328 960) sind Alkyl(meth)acrylate, einschließlich der Cycloalkyl(meth)acrylate, Aralkyl(meth)-acrylate und 2-Hydroxyalkyl(meth)acrylate, beispielsweise Hydroxypropylmethacrylat, Hydroxyethylmethacrylat, lsobornyl-acrylat, Isobornylmethacrylat, Butylglycolmethacrylat, Acetylglykolmethacrylat, Triethylenglycoldimethacrylat, Polyethylenglycoldimethacrylat, 2-Phenylethylmeth-acrylat, 2-Ethylhexylmethacrylat, Cyclohexylmethacrylat, Laurylmethacrylat und Hexandioldi(meth)acrylat. Verwendet werden können auch die langkettigen Monomere, wie sie in der US-A-3 066 112 beschrieben sind, auf der Basis von Bisphenol-A und Glycidylmethacrylat oder deren durch Addition von Isocyanaten entstandene Derivate. Geeignet sind auch Verbindungen des Typs Bisphenyl-A-diethyloxy(meth)acrylat und Bisphenol-A-dipropyloxy(meth)acrylat. Weiterhin können die oligoethoxylierten und oligopropoxylierten Bisphenol-A-diacryl- und -dimethacrylsäureester Verwendung finden. Gut geeignet sind außerdem die in der DE-A-2 816 823 genannten Diacryl- und Dimethacrylsäureester des Bis(hydroxymethyl)-tricyclo[5.2.1.0^{2.6}]-decans und die Diacryl- und Dimethacryl-säureester der mit 1 bis 3 Ethylenoxid- und/oder Propylenoxideinheiten verlängerten Verbindungen des Bis(hydroxymethyl)-tricyclo [5.2.1.0^{2,6}]-decans. Es können auch Mischungen der genannten Monomeren verwendet werden. Besonders geeignet sind Methacrylate, wie Bis-GMA, HEMA, TEGDMA, Plex 6661 und saure Methacrylate, wie 4-META.

Geeignete Füllstoffe sind beispielsweise Aerosile (Aerosil 200, COK 84 von Degussa), Quarze, Gläser insbesondere desaktivierte bzw. nicht basische Gläser, Fällungskieselsäuren (HDKH).

Geeignete Stabilisatoren umfassen Radikalfänger, wie substituierte oder nichtsubstituierte Hydroxyaromaten, insbesondere Methoxyphenol oder Jonol, HALS (Hinderd Amine Light Stabilizers), Schwermetallfänger, wie EDTA und lösliche Sulfate.

Bei den ionenabgebenden Substanzen sind solche bevorzugt, die die Freisetzung von Fluoridionen ermöglichen, wie Fluoridsalze der ersten oder zweiten Hauptgruppe, wie Natriumfluorid oder Calciumfluorid, oder komplexe Fluoridsalze, wie KZnF₃, oder wie sie in der EP-A-0 717 977 beschrieben werden, Fluorid abgebende Gläser sowie Mischungen dieser Fluroridionenquellen.

Als bakterizid oder antibiotisch wirksame Substanzen können beispielsweise Chlorhexidin, Pyridinumsalze oder die üblichen pharmazeutischen Substanzen, wie β-Lactamantibiotika (Penicilline), Cephalosporine, Tetracycline, Ansamycine, Kanamycine, Chloramphenicol, Fosfomycin, antibakterielle Makrolide, Polypeptid-Antibiotika, Chemotherapeutika, wie Sulfonamide, Dihydrofolatreduktase-Hemmstoffe, Nitrofuran-Derivate oder Gyrasehemmer verwendet werden.

Für die Wahl und Durchführung des aktivitätsterminierenden Mechanismus ist es im wesentlichen entscheidend, welches Enzym für die proteolytische Modifizierung des Kollagens verwendet wird.

Im folgenden werden einige bevorzugte Ausführungsformen genannt, die die Ausführung der Erfindung prinzipiell erklären.

Eine Möglichkeit die Erfindung auszuführen ist beispielsweise die Änderung des pH-Wertes der "Arbeitsumgebung" der Protease.

Verwendet man beispielsweise von Clotridium histolytikum eine Clostridiopeptidase A mit einem pH-Optimum um 7,0 so kann durch Absenken des pH-Wertes durch beispielsweise Zusatz von Säuren zu der verwendeten Mischung die Aktivität der verwendeten Protease beendet werden.

In einer anderen Ausführungsform kann das Enzym beispielsweise in bzw. mit einem neutralen Primer aufgetragen werden und wird nach der notwendigen Einwirkzeit mit dem sauren Bond überschichtet.

Eine weitere Ausführungsform liegt beispielsweise im Zusatz von säurebildenden Photopolymerisationsinitiatoren, wie sie in der WO-98/47046 oder in der DE-A-197 36 471 und DE-A-197 43 564 beschrieben sind. Diese werden durch einen Belichtungsschritt aktiviert und sorgen so für eine Absenkung des pH-Wertes.

Werden säuresensible Proteasen verwendet, so sollte die zur Aktivitätsterminierung notwendige pH-Absenkung, ausgehend vom pH-Optimum der jeweiligen Protease oder Proteasegemisches, soweit erfolgen, bis die Aktivitätsterminierung erreicht ist.

Dies kann in einer, zwei, drei, vier, fünf, sechs, sieben oder acht pH-Stufen erreicht werden. Welche pH-Stufen bevorzugt, bzw. besonders bevorzugt sind, hängt von der jeweilig zur Anwendung gebrachten Protease oder Proteasemischung ab (A. J. Barrett, N. D. Rawlings, J. F. Woessner, Handbook of Proteolytic Enzymes, Academic Press, San Diego, 1998).

Andererseits kann man auch beispielsweise bei saurem pH-Milieu arbeitende Enzyme wie Pepsin verwenden. Da diese bei einem pH-Wert von 1 bis 5 arbeiten, können sie direkt in der sauren Bondingrezeptur eingesetzt werden.

Die "Abschaltung" der Proteaseaktivität erfolgt durch Neutralisation der sauren Komponenten im Bonding durch den Hydroxylapatit-Anteil des Dentins. Der pH-Wechsel führt hier einerseits zu einer Aktivitätsterminierung des Pepsins und andererseits wird Pepsin bei einem pH größer pH 7 irreversibel inaktiviert (A. J. Barrett, N. D. Rawlings, J. F. Woessner, Handbook of Proteolytic Enzymes, Academic Press, San Diego, 1998, S. 810.

Bei dieser Art der Ausführungsform der Erfindung ist bei der Verwendung einer definierten Protease ableitbar, welcher Art die pH-Wertänderung sein muss, um die Aktivitätsterminierung zu erreichen.

Eine weitere Möglichkeit der Erfindungsausführung liegt in der örtlichen Begrenzung der Proteasewirkung. Dies kann beispielsweise in der Derivatisierung der Protease mit großen Molekülen wie Agarose (z.B. Sigma-Aldrich, Deisenhofen, Katalog 1999, P3286) oder durch Kopplung der Proteasen an in Bonding allgemein verwendeten Füllstoffen wie Quarze, Gläser oder Kieselgele (Herstellung der Enzymderivate beispielsweise nach J. Chem. Technol. Biotechnol., 1992, 54 (3), 215 -21) erfolgen. Prinzipiell ist jede Derivatisierung geeignet, die die Diffusion der Protease herabsetzt.

Die Herabsetzung der Diffusion kann, muss aber nicht, mit einem aktivitätsterminierenden Mechansimus kombiniert werden. Eine proteolytisch aktive Protease kann in der Hybridschicht oder im Haftverbund zwischen Zahnhartsubstanz und Dentalmaterial verbleiben, wenn durch eine Diffusionshemmung sichergestellt wird, dass die Protease mit seiner proteolytischen Aktivität nicht in tiefere Schichten vordringen kann. Vorteilhaft ist es wenn die Protease daran gehindert wird, unkontrolliert in tiefere Schichten der Zahnhartsubstanz vorzudringen.

Eine andere Möglichkeit der ortsspezifischen Fixierung der verwendeten Proteasen ist der Einbau der Proteasen in die Polymermatrix des Primers, und/oder des Bondes.

Beispielsweise ist hier die Derivatisierung mit Monomermolekülen, die fähig sind, mit den Molekülen der Primer oder/und Bondingmatrix chemische Bindung einzugehen, möglich. Dies kann in radikalisch polymerisiernden Systemen, beispielsweise durch Modifizierung der Proteasen mit Methacrylsäurederivaten, erreicht werden.

Es ist aber auch eine Addition oder Kondensation über funktionelle Gruppen der Proteasen selbst, beispielsweise über NH₂, SH, COOH, oder C-C-Doppelbindungen denkbar. Bei kationischen Systemen ist beispielsweise auch die Addition von beispielsweise NH₂- oder SH-Gruppen direkt an ein Epoxidmonomer der Matrix möglich, so dass hierfür nicht einmal eine Derivatisierung der eigentlichen Protease notwendig ist. Vorteilhaft ist es, wenn die Protease chemisch in die Polymermatrix eingebunden wird.

Die ortspezifische Fixierung der Protease durch chemische Einbindung in die Polymermatrix kann, muss aber nicht, mit einem aktivitätsterminierenden Mechanismus kombiniert werden. Eine proteolytisch aktive Protease kann in der Hybridschicht oder im Haftverbund zwischen Zahnhartsubstanz und Dentalmaterial verbleiben, wenn durch die chemische Einbindung in die Polymermatrix sichergestellt ist, dass die Protease mit seiner proteolytischen Aktivität nicht in tiefere Schichten vordringen kann. Vorteilhaft ist es, wenn die Protease daran gehindert wird, unkontrolliert in tiefere Schichten der Zahnhartsubstanz vorzudringen.

Eine weitere nicht erfindungsgemäße Möglichkeit die Aktivitätsterminierung der verwendeten Proteasen durchzuführen liegt in einer kurzzeitigen, für die Zahnhartsubstanz nicht schädlichen Erhöhung der Temperatur. Dies kann beispielsweise durch Zuführung von Energie aus einer externen Quelle (beispielsweise übliche Wärmequellen, übliche verwendet Polymerisationslampen, wie Elipar Trilight (Fa. Espe, Seefeld), aber auch Plasmalampen oder Laser oder elektromagnetische Strahler, oder Ultraschall, aber auch durch Erzeugung von chemischer Energie, wie die Polymerisationswärme eines nachträglich aufgebrachten Füllungsmaterials selbst erfolgen.

Eine andere Möglichkeit der Aktivitätsterminierung liegt in dem Zusatz von redoxaktiven Substanzen. Diese werden beispielsweise durch Belichtung des Primers/Bondings/Versieglers aktiviert und zerstören dann die Protease auf chemischem Wege. Als Beispiele für diese redoxaktiven Substanzen seien Radikalbildner wie übliche radikalbildende Initiatoren genannt. Diese sind in der Literatur beschrieben (z. B. J.-P Fouassier, Photoinitiation, Photopolymerization and Photocuring, Hanser Publishers, Munich, Vienna, New York, 1995 oder auch J.-P Fouassier, J. F. Rabek (Hrsg.), Radiation Curing in Polymer Science and Technology, Vol. II, Elsevier Apllied Science, London, New York, 1993). Diese können durch UV- oder sichtbares Licht aktivierbar sein, wie beispielsweise Benzoinalkylether, Benzilketale, Acylphosphinoxide oder aliphatische und aromatische 1,2-Diketonverbindungen, beispielsweise Campherchinon, wobei die Katalysatoraktivität durch Zusatz von Aktivatoren, wie tertiären Aminen oder organischen Phosphiten, in an sich bekannter Weise beschleunigt werden kann.

Geeignete Initiatorsysteme zur Auslösung der radikalischen Polymerisation über einen Redox-Mechanismus sind beispielsweise die Systeme Peroxid/Amin, Peroxid/Barbitursäurederivate oder Peroxid/Säuren. Bei Verwendung solcher Initiatorsysteme ist es zweckmäßig, einen Initiator (z. B. Peroxid) und eine Katalysatorkomponente (z. B. Amin) getrennt bereitzuhalten. Die beiden Komponenten werden dann kurz vor ihrer Anwendung miteinander homogen vermischt.

Eine weitere Möglichkeit der Aktivitätsterminierung liegt in der Hemmung von enzymaktivitätsentscheidenden Gruppen, Substanzen oder Cofaktoren.

Beispielsweise können die Metalle von Metallopeptidasen durch beispielsweise übliche Komplexbildner, wie Chelatisierungsmittel, wie EDTA oder seine Salze, komplexiert werden. Bei dieser Ausführungsform der Erfindung kommt es darauf an, das die Affinität des zugesetzten Komplexbildners zum Targetmetall höher als die der Protease zu dem Metall ist. Bevorzugt sind Komplexbildner mit einer hohen Affinität gegenüber bivalenten Kationen, besonders bevorzugt solche mit einer hohen Affinität gegenüber Zink, Cobalt, Eisen oder Mangan. Die Komplexbildner werden mindestens in äquimolaren Mengen eingesetzt. Enthalten die verwendeten Metallopeptidasen zwei, cokatalytisch wirkenden Metallionen (Cobalt und Mangan basierende und einige der Zink basierenden Metallopeptidasen), so können die Komplexbildner zur besseren Wirkung auch im Molverhältnis zwei zu eins berechnet auf Metallionen eingesetzt werden.

Eine weitere Möglichkeit zur Inhibierung dieser Proteasen liegt in der Zugabe von Ionen die schwerlösliche Salze mit den genannten Ionen bilden, wie Hydroxy-, Sulfid-, Phosphat-, Sulfat- oder Carbonationen.

Eine andere Aktivitätsterminierung ist bei solchen Proteasen möglich, deren katalytische Aktivität von Thiolgruppen oder Hydroxylgruppen abhängig ist. Hier ist es möglich, durch übliche Oxidationsmittel oder durch kovalente Modifikation z.B. Alkylierungsmittel, eine Aktivitätsterminierung der proteolytischen Aktivität der Proteasen zu erreichen.

Auch ist es möglich, eine Aktivitätsterminierung durch Zugabe von kommerziell erhältlichen Proteaseinhibitoren bzw. deren Mischungen, wie Pepstatin A (Fa. Sigma-Aldrich, Deisenhofen, Katalog 1999) zu erreichen.

In dieser Ausführungsform der Erfindung wird die Proteasehemmer-Mischung nach ausreichender Einwirkzeit der verwendeten Protease, bzw. der Proteasemischung mit der Protease bzw. Proteasemischung in Kontakt gebracht und ebenfalls auf der Zahnhartsubstanz belassen.

Eine weitere Möglichkeit zur Aktivitätsminimierung ist der nachgeschaltete Einsatz eines weiteren Enzyms, das nicht in der Lage ist, Kollagen zu zerschneiden oder modifizieren. Dieses weitere Enzym muss dann in der Lage sein, das kollagenabbauende bzw. -modifizierende Enzym zu desaktivieren.

Proteasen können entweder in einem separaten Schritt aufgetragen oder bevorzugt direkt in den Versieglern oder Bondings bzw. auch Primern oder Conditionem eingesetzt werden. Selbstverständlich ist auch eine Kombination der oben gezeigten Ausführungsformen möglich. Ebenfalls kann auch eine chemische und/oder physikalische Aktivitätsterminierung im Sinne einer Denaturierung des Enzyms durch allgemein bekannte Methoden wie z.B. Säurefällung, Aussalzen, oder Koagulation durch Schwermetallzusatz möglich.

Werden die Proteasen in einer separaten Lösung eingesetzt, so haben die Lösungen beispielsweise eine Konzentration von 0,001 mg/ml bis 10 mg/ml bevorzugt von 0,001 mg/ml bis 1 mg/ml besonders bevorzugt von 0.01 mg/ml bis 0,2 mg/ml.

Als Lösungsmittel können alle wässrigen und organischen Lösungsmittel eingesetzt werden, die die Aktivität der Enzyme nicht soweit beeinträchtigen, dass deren erfindungsgemäße Einsatz unmöglich gemacht wird. Bevorzugt sind wässrige Lösungen mit oder ohne Puffersysteme wie entionisiertes Wasser, Phosphat-Puffer, Tris-Puffer, und Glycin-Puffer. Geeignet sind auch Lösungsmittel, wie Dialkylketone (z. B. Aceton, Methyl-ethylketon), Acetylaceton oder Alkohole (z. B. Ethanol, Propanol) oder auch dünnfließende polymerisierbare Substanzen wie 2-Hydroxyethylmethacrylat oder (2,3-Epoxypropyl)-methacrylat sowie Mischungen davon.

Werden die Enzyme direkt im Versiegier oder im Bonding eingesetzt, so haben die Lösungen beispielsweise eine Konzentration von 0,001 mg/ml bis 20 mg/ml bevorzugt von 0,001 mg/ml bis 1 mg/ml besonders bevorzugt von 0.01 mg/ml bis 0,2 mg/ml.

Im folgenden wird die Erfindung anhand von Beispielen näher beschrieben.

### Vorbereitung der Rinderzähne

Pro Versuch werden fünf nach dem Extrahieren tiefgefrorene Rinderzähne aufgetaut, von restlichem Zahnfleisch gereinigt und die Wurzel durch Absägen mit einer Diamantsäge abgetrennt. Die noch verbleibende Pulpa wird mit Hilfe einer Pulpanadel entfernt und die Zähne dann mit Leitungswasser gespült. Plane Dentinoberflächen werden durch labiales Schleifen der Zähne an einer wassergekühlten Diamantschleifmaschine erhalten. Die Zähne werden dann so in Silikon eingebettet, dass die abgeschliffene, gut feucht gehaltene Oberfläche nach oben zeigt und erst dann noch mal mit einem feinen Siliziumcarbidschleifpapier nachgearbeitet. Dann wird auf jeden Zahn ein Wachsplättchen aufgeklebt, welches eine Ausstanzung von 6 mm Durchmesser hat (Prüffeld). Die so erhaltenen Prüfkörper werden in Anlehnung an das praxisübliche Vorgehen ("All-etch-Technik") mittels einer üblichen Phosphorsäurelösung (Ätzgel Minitip^{®}, Fa. ESPE Dental AG, Seefeld) für 20 Sekunden angeätzt und anschließend mit Wasser gründlich abgespült.

### Ausführungsbeispiel 1

### Aktivitätsterminierung durch pH-Änderung

Rinderzähne wurden entsprechend dem oben beschriebenen Verfahren vorbereitet. Anschließend wurden in einer wässrigen Lösung 20 µg Kollagenase aus Clostridium histolytikum Typ la (Fa. Sigma-Aldrich, Deisenhofen, Katalog 1999) in 5 µl entionisierten Wasser in das Prüffeld aufgebracht und gleichmäßig verteilt. Nach fünfminütiger Inkubation wurde das saure Pertac Universal Bond (Fa. ESPE Dental AG, Seefeld) nach Herstellervorschrift aufgebracht und nach Herstellervorschrift ausgehärtet. Anschließend wurde die Bondingschicht mit 50 µl entionisierten Wasser überschichtet. Nach einer Stunde wurden 40 µl von der Probe auf der Bondingschicht mit einer Pipette aufgenommen und zu einer Reagenzlösung zur Bestimmung der Kollagenase-Aktivität gegeben. In 960 µl Dinatriumhydrogenphosphat-Lösung (pH 7,0; 100 mM) befand sich 50 µg fluoreszenzmarkierte Gelatine (Fa. Molecular Probes, Göttingen). Die Gegenwart von Kollagenase-Aktivität ist an der Zunahme der Fluoreszenz beobachtbar. Die Anregungswellenlänge war nach Herstellervorschrift 495 nm und die Fluoreszenzemission wurde nach Herstellervorschrift bei 515 nm in einem Fluoreszenzphotometer der Fa. Kontron (SFM 25) gemessen. Es konnte keine Kollagenase-Aktivität gemessen werden.

Durch Überschichten der Kollagenase mit einem sauren Bonding wird die Aktivität des Enzyms, das ein pH-Optimum im Bereich von pH 7 aufweist, deaktiviert.

In einem Kontrollversuch wurden 20 µg Kollagenase in 50 µl Dinatriumhydrogenphosphat-Lösung (pH 7,0; 100 mM) auf eine ausgehärtete Bondingschicht aufgebracht. Nach einer Stunde wurde die Kollagenase-Aktivität bestimmt. Innerhalb einer Minute nahm die relative Fluoreszenz um über 100 % zu. In einem weiteren Kontrollexperiment wurde die ausgehärtete Bondingschicht direkt mit 50 µl der fiuoreszenzmarkierten Geiatine-Lösung überschichtet. Es konnte keine Kollagenase-Aktivität nachgewiesen werden.

Die durch das Pertac Universal Bond bedingte Versauerung hat die Kollagenase-Aktivität terminiert.

### Ausführungsbeispiel 2

### Aktivitätsterminierung durch pH-Änderung

2,5 ml einer Pepsinlösung (10 µg/ml) in einem 20 mM Dinatriumhydrogenphosphatpuffer (pH 2,0) wurden zu 1 g gemahlenem Rinderdentin gegeben und dort unter Schütteln für 20 min belassen. Anschließend wurde die Mischung für 1 min in einer Heraeus Biofuge Pico bei 10.000 rpm zentrifugiert. 900 µl des Überstandes wurden mit 12 µg fluoreszenzmarkiertem Casein (Fa. Molecular Probes, Göttingen) in 100 µl 20 mM Dinatriumhydrogenphosphatpuffer (pH 2,0) versetzt. Die Gegenwart proteolytischer Pepsinaktivität zeigt sich an der Zunahme der Fluoreszenz bei einer Anregungswellenlänge von 480 nm und bei einer Emissionswellenlänge von 510 nm. Die Messungen zeigten, dass durch die alkalisierende Wirkung des Dentins der pH soweit ins Alkalische verschoben wurde, so dass keine Pepsinaktivität mehr feststellbar war.

### Ausführungsbeispiel 3

### Aktivitätsterminierung durch Diffusionshemmung

Es wurde Agarose gekoppeltes Pepsin verwendet, kommerziell erhältlich bei der Fa. Sigma-Aldrich, Deisenhofen. Hiervon wurden 10 µg in die wässrige Phase von Prompt L-Pop (ESPE Dental AG, Seefeld) gegeben. Nach Herstellervorschrift wurde die wässrige Phase mit der zweiten Komponente des Bondings vermischt. Die Mischung wurde in eine 96er-Mikrotiterplatte überführt und mit einer Elipar Highlight Beleuchtungseinheit (Fa. ESPE Dental AG, Seefeld) durch Lichtpolymerisation (40 sec) ausgehärtet.

Die Bondingschicht wurde mit 100 µl Dinatriumhydrogenphosphatpuffer (50 mM, pH 2,0), enthaltend 5 µg fluoresenzmarkiertes Casein (Fa. Molecular Probes) überschichtet. Nach 30 min wurde mit einer Pipette 90 µl von der Bondingoberfläche entnommen und mit 110 µl des Dinatriumhydrogenphosphatpuffers vermischt. Die Fluoreszenzzunahme wurde bei 510 nm Emission (Anregung bei 480 nm) gemessen. Die Zunahme der relativen Fluoreszens um 30 % zeigte an, dass an der Oberfläche der Bondingschicht proteolytisch aktives Pepsin vorlag.

Es wird somit gezeigt, dass das Enzym aktiv bleiben kann, aber die Mobilität des Enzyms durch Kopplung an Agarose eingeschränkt wird und das Enzym somit nicht in tiefere Schichten des Dentins wandern kann.

In einem Kontrollversuch wurde die ausgehärtete Pepsin/Bondingschicht (Prompt-L-Pop, ESPE Dental AG, Seefeld) mit 100 µl Dinatriumhydrogenphosphatpuffer (50 mM. pH 2,0) überschichtet. Nach 30 min wurde mit einer Pipette 90 µl von der Bondingoberfläche entnommen und mit 110 µl des Dinatriumhydrogenphophatpuffer enthaltend 5 µg fluoreszenzmarkiertes Casein (Fa. Molecular Probes) vermischt. Die relative Fluoreszenz wurde bei 510 nm Emission (Anregung bei 480 nm) bestimmt. Im Vergleich zur Kontrollprobe, die 5 µg fluoreszenzmarkiertes Casein in 200 µl Meßlösung enthielt, konnte keine Fluoreszenzzunahme beobachtet werden. Dies belegt, dass an Agarose gekoppeltes Enzym die ausgehärtete Bondingschicht nicht verlassen kann.

### Ausführungsbeispiel 4

Die Vorbereitung der Rinderzähne und der Prüffelder auf den Rinderzähnen wurde unter "Vorbereitung Rinderzähne" beschrieben. EBS-Multi der Fa. ESPE Dental AG, Seefeld beinhaltet neben einem Ätzgel, eine Primer-Lösung und eine Bonding-Lösung. In 1 ml Primer-Lösung wurde 1 mg Kollagenase aus Clostridium histolytikum Typ Ia (Fa. Sigma-Aldrich, Deisenhofen Katalog 1999) gelöst. In das Prüffeld wurde nach Gebrauchsanweisung das Bonding EBS-Multi eingebracht, wobei vergleichend der Primer mit Kollagenase und ohne Kollagenase verwendet wurde. Anschließend wurde nach der Gebrauchsanweisung des Herstellers eine Füllung Pertac (Fa. ESPE Dental AG, Seefeld) eingebracht. Nach 24-stündiger Lagerung bei 36°C und 100% Luftfeuchtigkeit wurden die Wachsplättchen entfernt und die Prüfkörper in einem Zugversuch (Zwick-Universalprüfmaschine) abgezogen.

Der durchschnittliche Haftwert von 5 Rinderzähnen, der mit der Primer-Variante ohne Kollagenase erzielt wurde, betrug 3,96 MPa mit einer Standardabweichung von 31 %. Der durchschnittliche Haftwert der mit der Primer-Variante mit Kollagenase erzielt wurde, betrug 10,5 MPa mit einer Standardabweichung von 8,7 %.

Durch Integration der Kollagenase in den ein Primer-System konnten somit signifikant die Haftwerte erhöht und die Standardabweichung der Haftwerte verringert werden.

### Ausführungsbeispiel 5

Die Vorbereitung der Rinderzähne und der Prüffelder auf den Rinderzähnen wurde unter "Vorbereitung Rinderzähne" beschrieben. Prompt L-Pop (Fa. ESPE Dental AG, Seefeld) besteht aus einer wässrigen Phase und einer Polymermonomerphase, die in einer Blisterverpackung in zwei getrennten Kammern vorgehalten werden.

Aus einer Reihe von Blistern wurden die Flüssigkeiten entnommen. Die wässrige Phase wurde mit Pepsin bis zu einer Konzentration von 5 mg pro ml angereichert. Wässrige Phase und Polymermonomerphase wurden in einem Verhältnis von 1:5 gemischt, so dass die Endkonzentration von Pepsin 1 mg pro ml Bonding erreicht wurde.

In das Prüffeld wurde nach Gebrauchsanweisung das Bonding Prompt L-Pop eingebracht, wobei vergleichend die wässrige Phase einmal mit und einmal ohne Pepsin-Zusatz verwendet wurde. Anschließend wurde nach der Gebrauchsanweisung des Herstellers eine Füllung Hytac (Fa. ESPE Dental AG, Seefeld) eingebracht. Nach 24-stündiger Lagerung bei 36°C und 100% Luftfeuchtigkeit wurden die Wachsplättchen entfernt und die Prüfkörper in einem Zugversuch (Zwick-Universalprüfmaschine) abgezogen.

Der durchschnittliche Haftwert von 5 Rinderzähnen, der mit der Prompt L-Pop Variante ohne Pepsin erzielt wurde, betrug 4,1 MPa mit einer Standardabweichung von 39 %. Der durchschnittliche Haftwert von 5 Rinderzähnen, der mit der Prompt L-Pop Variante mit Pepsin erzielt wurde, betrug 5,9 MPa mit einer Standardabweichung von 9 %.

Durch Integration von Pepsin in ein Bonding konnten somit signifikant die Haftwerte erhöht und die Standardabweichung der Haftwerte erniedrigt werden.

### Vergleichsbeispiele:

Im ersten Versuch wurden die Rinderzähne ohne weitere Behandlungsschritte dem unten beschriebenen Bonding- und Füllungsverfahren unterzogen. Es erfolgte dann die Überprüfung des Haftverbundes erfolgt durch einen Haftabzugsversuch mit einer Zwick-Universalprüfmschiene.

Im zweiten Versuch wurden die Rinderzähne mit einer Kollagenaselösung behandelt. Eingesetzt wurde eine kommerziell erhältliche Kollagenase aus Clostridium histolytikum Typ la (Fa. Sigma-Aldrich, Deisenhofen, Katalog 1999). Auf jeden Rinderzahn wurden jeweils 50 µg Kollagenase in 50 µl Dinatriumhydrogenphosphatpuffer (pH 7,0; 50 mM) mittels Pipette in das Prüffeld aufgebracht. Nach fünf-minütiger Inkubation wird die Kollagenase-Lösung gründlich abgespült. Es folgt das unten beschriebene Bonding- und Füllungsverfahren und dann die Überprüfung des Haftverbundes durch einen Haftabzugsversuch mit einer Zwick-Universalprüfmaschiene

Im dritten Versuch werden die Rinderzähne nach dem selben Verfahren behandelt wie im zweiten Versuch beschrieben. Diesmal beträgt die Inkubationszeit 25 min.

### Bonding-und Füllungsverfahren

In das Prüffeld wurde nach Gebrauchsanweisung das Bonding EBS-Multi^{®} (Fa. ESPE Dental AG, Seefeld) eingebracht und anschließend wurde nach Gebrauchsanweisung des Herstellers eine Füllung mit Hytac^{®} (Fa. ESPE Dental AG, Seefeld) eingebracht. Nach 24-stündiger Lagerung bei 36°C und 100% Luftfeuchtigkeit wurden die Wachsplättchen entfernt und die Prüfkörper in einem Zugversuch (Zwick-Universalprüfmaschine) abgezogen. Die dabei ermittelten durchschnittlichen Dentinhaftwerte von jeweils fünf Rinderzähnen sind Abbildung 1 zu entnehmen.

### Legende zu Abbildung 1

Auf der Y-Achse werden die ermittelten durchschnittlichen Haftwerte der Haftversuche auf Rinderdetin in MPa angegeben. Die X-Achse wird entsprechend den drei durchgeführten Versuchen nach Inkubationszeiten mit der Kollagenase-Lösung unterteilt. 0 min: keine Kollagenase-Inkubation; 5 min: Kollagenase-Inkubation für 5 min; 25 min: Kollagenase-Inkubation für 25 min. Unter jedem Ergebnisbalken ist eine REM-Aufnahme der bei der Behandlung entstehenden Dentinoberfläche abgebildet.

### Röntgenelektronenmikroskopische Untersuchung

Jeweils ein Rinderzahn wurde nach dem für die Versuche eins, zwei und drei beschriebenen Verfahren behandelt. Das Bonding- und Füllungsverfahren wurden nicht durchgeführt. Die Zähne wurden getrocknet und mit einem Gerät der Fa. Balzers Union bespattert. Anschließend wurden die behandelten Rinderdentinoberflächen mit einem REM-Gerät der Fa. Hitachi mikroskopisch untersucht. Abbildungen der Oberflächen sind in Abbildung 1 in einer 1000-fachen Vergrößerung zu sehen.

## Patentansprüche

1. Oberfächenbehandlungsmittel für dentales Hartgewebe zur Modifizierung der nach der Präparation oder dem Ätzen vorhandenen Kollagenschicht, umfassend ein Enzym und ein die Enzymaktivität begrenzendes Mittel, wobei das oder die Enzyme gewählt sind aus der Gruppe der Peptidasen, Peptidylpeptidasen, Dipetidasen, Dipeptidylpeptidasen, Oligopeptidasen, Proteinasen, Endopeptidasen, Exopeptidasen, Serinpeptidasen, Cysteinpeptidasen, Aspartatpeptidasen, Metallopeptidasen und wobei das die Enzymaktivität begrenzende Mittel gewählt ist aus Säuren, Basen, Puffern, polymerisierbare Substanzen, Salzen, oxidierbaren oder reduzierbaren Stoffen, Proteasehemmern, nicht-kollagen modifizierenden Enzymen und/oder diffusionshemmenden Stoffen.

2. Oberflächenbehandlungsmittel nach Anspruch1, enthaltend mindestens eine Substanz gewählt aus: Füllstoffe, Farbstoffe, Fließmodifikatoren, Stabilisatoren, Initiatoren, Lösungsmittel, ionenabgebende Substanzen, bakterizid oder antibiotisch wirksame Substanzen, die Röntgenopazität erhöhende Verbindungen, polymerisierbare Substanzen, radikalbildende Initiatoren.

3. Kit, enthaltend ein Oberflächenbehandlungsmittel nach einem der vorstehenden Ansprüche und mindestens einen Bestandteil gewählt aus: Bondingmaterial, Primer, Säure, weiteres Zahnkonditionierungsmittel und/oder Füllungsmaterial.

4. Verwendung von die Enzymaktivität begrenzenden Mitteln zur Herstellung eines Oberflächenbehandlungsmittels enthaltend ein Enzym zur Modifizierung der nach der Präparation oder dem Ätzen vorhandenen Kollagenschicht für ein Verfahren zur Oberflächenbehandlung von dentalem Hartgewebe, wobei das enzymhaltige Mittel auf dem Hartgewebe verbleibt, und wobei das die Enzymaktivität begrenzende Mittel gewählt ist aus: Säuren, Basen, Puffern, polymerisierbare Substanzen, Salzen, oxidierbaren oder reduzierbaren Stoffen, Proteasehemmern, nichtkollagenmodifizierenden Enzymen und/oder diffusionshemmenden Stoffen und wobei das oder die Enzyme gewählt sind aus: der Gruppe der Peptidasen, Peptidylpeptidasen, Dipetidasen, Dipeptidylpeptidasen, Oligopeptidasen, Proteinasen, Endopeptidasen, Exopeptidasen, Serinpeptidasen, Cysteinpeptidasen, Aspartatpeptidasen, Metallopeptidasen.

5. Verwendung nach Anspruch 4, wobei das Verfahren zur Oberflächenbehandlung das Aufbringen mindestens eines Materials umfasst, gewählt aus: Bonding, Primer, Conditioner, Füllungsmaterial.

6. Verwendung nach einem der Ansprüche 4 bis 5, wobei das Mittel vor der Oberflächenbehandlung durch Mischen eines Enzyms und eines die Enzymaktivität begrenzenden Mittels hergestellt wird.

7. Verwendung nach einem der Ansprüche 4 bis 6 umfassend die Schritte:
a) Auftragen eines Oberflächenbehandlungsmittels, enthaltend ein Enzym und gegebenenfalls ein die Enzymaktivität begrenzendes Mittel, b)Überschichten des Mittels aus Schritt a) mit einem Material gewählt aus: Bondingmaterial, Primer, Säure, Zahnkonditionierungsmittel und/oder Füllungsmaterial, wobei das Oberflächenbehandlungsmittel auf dem Hartgewebe verbleibt und die Aktivität des Enzyms durch das die Enzymaktivität begrenzende Mittel aus Schritt a) und/oder durch das Überschichten des Mittels aus Schritt a) mit dem Material aus Schritt b) begrenzt wird.

8. Verwendung nach Anspruch 7, wobei die Aktivität des Enzyms begrenzt wird durch die Änderung des pH-Wertes, der Salzkonzentration des Redoxpotentials, Zusatz von Proteasehemmern, Bindung des Enzyms an diffusionshemmende Stoffe, Einpolymerisation in diese und/oder den Zusatz von nicht kollagenmodifizierenden Enzymen.

## Claims

1. Surface treatment agent for hard dental tissue for modifying the existing collagen layer after preparation or etching, comprising an enzyme and an enzyme-activity-limiting agent, wherein the enzyme or the enzymes are selected from the group consisting of the peptidases, peptidyl peptidases, dipeptidases, dipeptidyl peptidases, oligopeptidases, proteinases, endopeptidases, exopeptidases, serine peptidases, cysteine peptidases, aspartate peptidases, metallopeptidases, and wherein the enzyme-activity-limiting agent is selected from the group consisting of acids, bases, buffers, polymerizable substances, salts, oxidizable or reducible materials, protease inhibitors, non-collagen-modifying enzymes and/or diffusion-inhibiting materials.

2. Surface treatment agent according to Claim 1, comprising at least one substance selected from: fillers, dyes, flow modifiers, stabilizers, initiators, solvents, ion donor substances, bactericidal or antibiotic substances, compounds which increase the X-ray opacity, polymerizable substances, free radical-forming initiators.

3. Kit comprising a surface treatment agent according to either one of the preceding claims and at least one constituent selected from: bonding material, primer, acid, further tooth conditioner and/or filling material.

4. Use of enzyme-activity-limiting agents for producing a surface treatment agent comprising an enzyme for modifying the existing collagen layer after preparation or etching for a method for surface treatment of hard dental tissue, wherein the enzyme-containing agent remains on the hard tissue, and wherein the enzyme-activity-limiting agent is selected from the group consisting of acids, bases, buffers, polymerizable substances, salts, oxidizable or reducible materials, protease inhibitors, non-collagen-modifying enzymes and/or diffusion-inhibiting materials, and wherein the enzyme or the enzymes are selected from the group consisting of the peptidases, peptidyl peptidases, dipeptidases, dipeptidyl peptidases, oligopeptidases, proteinases, endopeptidases, exopeptidases, serine peptidases, cysteine peptidases, aspartate peptidases, metallopeptidases.

5. Use according to Claim 4, wherein the method for surface treatment comprises applying at least one material selected from: bonding material, primer, conditioner, filling material.

6. Use according to either one of Claims 4 and 5, wherein the agent is produced before the surface treatment by mixing an enzyme and an enzyme-activity-limiting agent.

7. Use according to any one of Claims 4 to 6, comprising the steps: a) applying a surface treatment agent comprising an enzyme and optionally an enzyme-activity-limiting agent, b) adding as an overcoat to the agent from step a) a material selected from the group consisting of bonding material, primer, acid, tooth conditioner and filling material, wherein the surface treatment agent remains on the hard tissue and the activity of the enzyme is limited by the eaazyme-activity-limiting agent from step a) and/or by the addition of the material from step b) as an overcoat to the agent from step a).

8. Use according to Claim 7, wherein the activity of the enzyme is limited by the change in pH, in salt concentration, in the reduction potential, addition of protease inhibitors, binding of the enzyme to diffusion-inhibiting materials, polymerizational incorporation into these and/or the addition of non-collagen-modifying enzymes.

## Revendications

1. Agent de traitement de surface pour tissu dur dentaire en vue de la modification de la couche de collagène présente après la préparation ou la cautérisation, comprenant une enzyme et un agent limitant l'activité enzymatique, où la ou les enzymes est/sont choisi(e)s dans le groupe des peptidases, des peptidylpeptidases, des dipeptidases, des dipeptidylpeptidases, des oligopeptidases, des protéinases, des endopeptidases, des exopeptidases, des sérine-peptidases, des cystéine-peptidases, des aspartate-peptidases, des métallopeptidases et où l'agent limitant l'activité enzymatique est choisi parmi les acides, les bases, les tampons, les substances polymérisables, les sels, les substances oxydables ou réductibles, les inhibiteurs de protéase, les enzymes ne modifiant pas le collagène et/ou les substances inhibant la diffusion.

2. Agent de traitement de surface selon la revendication 1, contenant au moins une substance choisie parmi : les charges, les colorants, les modificateurs d'écoulement, les stabilisateurs, les initiateurs, les solvants, les substances libérant des ions, les substances bactéricides ou actives de manière antibiotique, les composés augmentant l'opacité aux rayons X, les substances polymérisables, les initiateurs formant des radicaux.

3. Kit, contenant un agent de traitement de surface selon l'une quelconque des revendications précédentes et au moins un constituant choisi parmi : les matériaux liants, les apprêtes, les acides, d'autres agents de conditionnement des dents et/ou les matériaux de remplissage.

4. Utilisation d'agents limitant l'activité enzymatique pour la préparation d'un agent de traitement de surface contenant une enzyme pour la modification de la couche de collagène présente après la préparation ou la cautérisation pour un procédé de traitement de surface de tissus durs dentaires, où l'agent contenant des enzymes reste sur le tissu dur et où l'agent limitant l'activité enzymatique est choisi parmi : les acides, les bases, les tampons, les substances polymérisables, les sels, les substances oxydables ou réductibles, les inhibiteurs de protéases, les enzymes ne modifiant pas le collagène et/ou les substances inhibant la diffusion et où la ou les enzymes est/sont choisi(e)s dans le groupe des peptidases, des peptidylpeptidases, des dipeptidases, des dipeptidylpeptidases, des oligopeptidases, des protéinases, des endopeptidases, des exopeptidases, des sérine-peptidases, des cystéinepeptidases, des aspartate-peptidases, des métallopeptidases.

5. Utilisation selon la revendication 4, où le procédé pour le traitement de surface comprend l'application d'au moins un matériau, choisi parmi : un liant, un apprêt, un agent de conditionnement, un matériau de remplissage.

6. Utilisation selon l'une quelconque des revendications 4 à 5, où l'agent est préparé avant le traitement de surface par mélange d'une enzyme et d'un agent limitant l'activité enzymatique.

7. Utilisation selon l'une quelconque des revendications 4 à 6 comprenant les étapes :
a) application d'un agent de traitement de surface, contenant une enzyme et le cas échéant un agent limitant l'activité enzymatique,
b) recouvrement de l'agent de l'étape a) par un matériau choisi parmi : un matériau liant, un apprêt, un acide, un agent de conditionnement des dents et/ou un matériau de remplissage,
l'agent de traitement de surface restant sur le tissu dur et l'activité de l'enzyme étant limitée par l'agent limitant l'activité enzymatique de l'étape a) et/ou par le revêtement de l'agent de l'étape a) par le matériau de l'étape b).

8. Utilisation selon la revendication 7, où l'activité de l'enzyme est limitée par la modification du pH, la concentration en sel du potentiel redox, l'addition d'inhibiteurs de protéase, la liaison de l'enzyme sur des substances inhibant la diffusion, la copolymérisation dans celles-ci et/ou addition d'enzymes ne modifiant pas le collagène.
